# FASCICULE DE BREVET EUROPEEN

(11) **EP 0 856 299 B1**
(45) Date de publication et mention de la délivrance du brevet: **28.07.2004**
(21) Numéro de dépôt: 98400209.7
(22) Date de dépôt: 02.02.1998
(51) Int. Cl.: A61F 2/00, A61F 2/06, A61F 2/28

(54) **Prothèse métallique de soutien et/ou de remplacement tissulaire à porosité ouverte**
Metallische Prothese zur Unterstützung und/ oder zum Ersatz von offenzelligem Gewebe
Metallic prosthesis for the support and/or replacement of open cell tissue

(30) Priorité: 03.02.1997 FR 9701156
(43) Date de publication de la demande: 05.08.1998
(73) Titulaire: OFFICE NATIONAL D'ETUDES ET DE RECHERCHES AEROSPATIALES (ONERA), 92320 Chatillon sous Bagneux (FR)
(72) Inventeur: Walder, André, 94240 La Hay Les Roses (FR); Debry, Christian, 75015 Paris (FR)
(74) Mandataire: Warcoin, Jacques

(56) Documents cités:
- WO-A-90/06093
- US-A- 4 281 669
- US-A- 4 374 669
- US-A- 4 550 448

## Description

La présente invention concerne le domaine général des prothèses métalliques implantables chez l'homme, dont la porosité ouverte permet la colonisation par des cellules vivantes, jouant ainsi le rôle d'une structure support permettant la reconstruction naturelle d'un organe disparu à la suite d'une opération ou d'un choc traumatique.

Il peut en particulier s'agir de prothèses laryngées totales destinées à permettre la reconstitution d'un larynx chez des malades ayant subi une laryngectomie, i.e. une ablation totale du larynx par exemple à la suite d'un cancer.

De manière plus générale, le domaine de la présente invention s'étend à toutes les prothèses de reconstruction locale concernant une paroi osseuse détruite telle que la calotte crânienne, les mandibules, ou encore la paroi thoracique.

Dans l'état actuel de la technique, il n'existe pas de véritable larynx artificiel pour les malades laringectomisés. La seule méthode connue consiste après ablation d'une partie ou de la totalité du larynx, à séparer chirurgicalement les fonctions de déglutition et de respiration. Cette dernière fonction est alors assumée par un orifice de trachéotomie situé sur le devant du cou. Par ailleurs, la fonction de phonation est fortement entamée, car l'air de respiration ne passe plus par les cordes vocales qui ont été éliminées lors de l'opération.

A ce jour, on peut donc considérer qu'il n'existe pas à proprement parler de prothèse laryngée qui permettrait de remplacer l'organe disparu. Les malades sont au mieux munis d'une prothèse phonatoire ne supprimant pas l'orifice définitif de trachéotomie, mais permettant une rééducation vocale le plus souvent satisfaisante, et parfois d'une mini-prothèse extérieure qui camoufle l'orifice de la trachéotomie et aide à filtrer l'air, à le réchauffer et à l'humidifier, toutes fonctions normalement assurées par le nez.

Dans le cas de reconstructions crâniennes ou autres, il est fait appel à plusieurs techniques aujourd'hui considérées comme classiques, mais qui ne sont toutefois pas dépourvues d'inconvénients.

Il existe tout d'abord l'autogreffe, par exemple d'un prélèvement osseux. Le problème essentiel de cette technique d'autogreffe réside dans la vascularisation des greffons qui peuvent progressivement être éliminés par le milieu.

On peut également recourir à l'implantation d'une pièce en alliage de titane massif dont l'intégration dans les tissus n'est pas toujours parfaite, sans parler du poids excessif d'une telle prothèse.

La présente invention a précisément pour but la mise au point d'un procédé de fabrication de prothèses métalliques à porosité ouverte, de soutien et/ou de remplacement tissulaire, notamment destinées à l'implantation cervico-maxillo-faciale et, plus particulièrement, de prothèses de reconstitution laryngée.

L'état de la technique antérieure peut être illustré par un certain nombre de documents de brevets et en particulier,
1) Le brevet US n° 4 374 669 concerne des prothèses pour implants cardiovasculaires qui sont destinées à être implantées à l'intérieur du corps, sans, contact avec le milieu extérieur, c'est-à-dire dans des conditions aseptiques.
   Ce document de technique antérieure enseigne la réalisation d'une prothèse orthopédique massive sur laquelle est apporté un revêtement poreux à porosité ouverte, qui est destiné à favoriser l'accrochage du tissu osseux et l'incorporation de la prothèse dans le corps. Conformément à ce brevet, on fait croître sur le revêtement poreux à porosité ouverte et à l'intérieur de la masse de celui-ci, une ou plusieurs couches de cellules à partir de cellules du sang, permettant ainsi d'éviter des problèmes de thrombo-embolie par contact direct du sang avec la pièce support non poreuse.
2) Le brevet US n° 4 281 669, relatif à la réalisation d'une électrode de stimulateur cardiaque, contient rigoureusement le même enseignement que le document précédemment analysé.
3) Le brevet US n° 4 550 448 enseigne la réalisation de prothèses osseuses pour diverses applications telles que des prothèses du genou, os plats, ponts entre os, doigts, implants dentaires, bien entendu destinées à être implantées en milieu parfaitement aseptique.

Là encore il s'agit d'un revêtement métallique poreux à porosité ouverte (35 à 40%), composé de deux couches de particules de poudre sur un substrat métallique dense.

Tout comme dans les cas d'application des deux brevets précédemment analysés, il s'agit de favoriser l'accrochage du tissu osseux sur la prothèse en évitant l'utilisation d'un ciment.

La présente invention s'est attachée à améliorer la fabrication de ce type de prothèses métalliques à porosité ouverte comme définies dans les revendications annexées.

Conformément à la présente invention, ces prothèses métalliques, sont mises en forme, par solidarisation entre elles, de microsphères de titane ou d'alliage à base de titane ou d'alliages biocompatibles, lesdites microsphères étant placées dans un moule de forme appropriée, puis solidarisées entre elles par fusion locale au voisinage de leur point de contact par frittage par électro-étincelage.

Une telle prothèse métallique à porosité ouverte de soutien et de remplacement tissulaire doit pouvoir être soumise à une colonisation tissulaire complète. Il s'agit en fait d'une structure rigide constituant un véritable support de l'organe à reconstituer et destinée à venir au contact d'autres tissus vivants. Contrairement à d'autres prothèses qui sont destinées à être implantées dans un environnement aseptique, les prothèses métalliques, objet de la présente invention, présentent en raison de leur porosité une aptitude à la colonisation tissulaire rapide, les cellules vivantes créant entre elles un véritable réseau de maillage englobant les microsphères.

De même, en raison du choix des caractéristiques dimensionnelles et de l'état de surface desdites microsphères utilisées, les bactéries ne s'accrochent pas aux parois de la prothèse qui peut ainsi être implantée dans un environnement non aseptique.

Les microsphères entrant dans la composition du matériau de la prothèse doivent être le plus uniforme possible. Il s'est en effet avéré que l'accrochage bactérien, qu'il convient précisément d'éviter, est bien supérieur lorsque la surface des microsphères présente des aspérités ou imperfections résultant de leurs procédés de préparation. Dans pareilles situations, les bactéries peuvent se nicher dans ces aspérités et les colonies de bactéries se développent alors très rapidement entraînant à court terme une obturation des espaces entre les sphères et bloquant ainsi la colonisation cellulaire de la prothèse.

Dans le même ordre d'idée, les microsphères doivent être exemptes de satellites. En effet, il est impossible d'espérer une véritable colonisation cellulaire en profondeur dans le matériau de prothèse si d'emblée, l'accrochage bactérien favorisé par la présence de satellites ou d'excroissance à la surface des microsphères vient s'opposer à une telle colonisation tissulaire.

A priori, les microsphères de titane ou d'alliage à base de titane pourraient être obtenues sous la forme de poudre élaborée par un certain nombre de procédés différents.

Parmi ces procédés, on pourrait penser à retenir le procédé HDH, c'est-à-dire l'hydrogénation-déshydrogénation. Ce procédé consiste à soumettre un lingot d'alliage de titane dans une atmosphère d'hydrogène à une température où l'alliage absorbe le gaz ; c'est la phase d'hydrogénation. Le produit obtenu, très fragile, peut donc être réduit en forme de poudre par broyage. Dans une seconde étape, la poudre sous vide est portée à une haute température à laquelle l'hydrogène se trouve éliminé ; il s'agit de l'étape de déshydrogénation.

La poudre obtenue par la mise en oeuvre d'un tel procédé HDH est de forme très irrégulière en raison du broyage et il existe en plus un risque de pollution par le produit constituant la jarre. Au surplus, un tel procédé HDH risque d'entraîner un accroissement important de la teneur en oxygène de la poudre métallique ainsi produite. La forme irrégulière des particules présente des aspérités et des imperfections susceptibles de favoriser l'accrochage bactérien. Ce procédé se révèle donc inadapté pour la fabrication des prothèses, dans le cadre de la présente invention.

Contrairement aux alliages à base de nickel qui peuvent être également transformés par atomisation par gaz, les poudres d'alliage de titane ne peuvent pas être produites par cette technologie en raison de la grande réactivité de ce métal à l'égard des creusets céramiques utilisés. Quelques essais ont été tentés pour produire néanmoins des poudres avec un tel procédé, en utilisant des creusets revêtus d'oxydes de terres rares insolubles dans le titane. Ce procédé conduit à des poudres, en général d'une bonne sphéricité, mais présentant des satellites de petites tailles accrochés à leur surface.

Selon une caractéristique avantageuse de la présente invention, lesdites microsphères se présentent sous la forme d'une poudre qui est obtenue par un procédé de pulvérisation à l'électrode tournante ("Powder Metallurgy of Superalloys", G. H. GESSINGER, Butterworths Monographs in Materials, pages 29 à 32). Un tel procédé consiste à fondre, soit à l'aide d'un arc électrique ou d'une torche plasma sous gaz neutre, soit par un faisceau d'électrons sous vide, la surface frontale d'un cylindre en rotation rapide autour de son axe. Le liquide ainsi formé migre, sous l'action des forces d'inertie centrifuge, vers la périphérie du lingot en rotation, d'où il s'échappe sous forme de gouttelettes qui se refroidissent et se solidifient pendant leur vol dans le gaz neutre contenu dans l'enceinte. Leur diamètre est en relation avec la vitesse de rotation du lingot, avec une distribution granulométrique très étroite autour d'un diamètre moyen. On a observé dans la pratique que l'on pouvait ainsi obtenir des poudres dont les grains microsphèriques sont pratiquement parfaits, c'est-à-dire avec quasiment pas de relief de surface et sans satellite. De surcroît, leur composition reste rigoureusement identique à celle du lingot de départ.

Selon une caractéristique avantageuse de la présente invention, la prothèse métallique est obtenue à partir de microsphères qui présentent pratiquement une géométrie parfaitement sphérique, et/ou un état de surface pratiquement dépourvu de toutes irrégularités telles qu'aspérités ou satellites.

Selon une caractéristique avantageuse de la présente invention, les microsphères présentent une faible dispersion granulométrique. Avantageusement, elles ont une dimension granulométrique comprise entre 50 et 500 µm et de préférence entre 150 et 300 µm.

Dans le cas où le réglage de la distribution granulométrique, par adaptation de la seule vitesse de rotation du lingot, se trouve insuffisant dans la pratique, il est bien entendu possible de procéder à un calibrage supplémentaire des microsphères par une opération de tamisage approprié.

Il est essentiel de remarquer ici que, parmi les différents procédés a priori utilisables pour produire des poudres de microsphères de titane ou d'alliage à base de titane, seul le procédé à l'électrode tournante permet d'obtenir des poudres parfaitement sphériques et sans aspérité qui répondent aux exigences médicales et permettent éventuellement par un tri granulométrique supplémentaire de définir avec précision la taille de la porosité recherchée. De plus, le fait que le procédé maintienne la composition de l'alliage et n'apporte aucune pollution constitue une garantie du point de vue de la biocompatibilité et de la non toxicité du matériau de la prothèse.

Dans la présente invention, la poudre de microsphères métalliques utilisée peut être une poudre de métal de titane pur, d'un alliage à base de titane ou de toute autre base d'alliages possédant un caractère de biocompatibilité et de non toxicité. Parmi les alliages à base de titane, on mentionnera ceux présentant une proportion prépondérante de titane et pouvant contenir au moins un autre métal choisi parmi l'étain, le niobium, le palladium, le zirconium, le tantale, le chrome, l'or et le silicium. Parmi ces métaux complémentaires, il semble que le niobium, le tantale et le zirconium soient ceux permettant d'obtenir des alliages dotés de la meilleure biocompatibilité.

Il est essentiel de comprendre qu'il n'existe actuellement aucun matériau complètement biocompatible. On définit la biocompatibilité comme l'interaction entre les tissus vivants et l'hôte, aboutissant au mieux à un équilibre permettant le non rejet du matériau receveur. L'avantage du titane pur réside dans l'absence quasi complète de réaction des cellules vivantes à son égard.

Les prothèses métalliques, objet de la présente invention, résultent donc d'une opération de mise en forme par solidarisation entre elles des microsphères précédemment décrites. Une telle opération est obtenue par traitement thermique, dans un moule de forme appropriée.

Pareille opération de traitement thermique de mise en forme de la prothèse peut être réalisée de différentes manières. Il peut par exemple s'agir d'un frittage thermique sous vide. Une telle opération de frittage thermique sous vide nécessite l'utilisation d'enveloppes métalliques comportant l'empreinte des prothèses à réaliser. Dans la pratique, des essais ont montré que des enveloppes en acier doux permettaient un frittage jusqu'à 1 050° C en raison d'un eutectique Ti-Fe à 1 085°C, température ne conduisant pas nécessairement à une liaison satisfaisante des microsphères entre elles. Au delà de cette température, il est nécessaire d'utiliser une enveloppe en titane qui, après frittage, risque de poser des problèmes de séparation entre la pièce frittée et son enveloppe. Ces problèmes peuvent être surmontés par interposition d'une feuille mince d'un matériau réfractaire (Mo par exemple), mais d'une manière générale, le procédé est compliqué et coûteux.

L'opération de mise en forme par traitement thermique peut également être conduite de façon avantageuse par électro-étincelage ("Preforming using high-voltage electrical discharge", S. T. S. Al-Hassani & T. J. Davies, Powder Metallugy, 1980). Ce procédé permet d'utiliser des enveloppes, de natures variées telles que du verre, des matières plastiques, des céramiques, du carton, etc, dont la principale propriété requise est d'être électriquement isolante. Cette technique permet de fabriquer des pièces de forme très variées en contrôlant parfaitement les conditions de soudure entre les différentes microsphères entre elles. En effet, l'élévation de température globale de la poudre est de l'ordre de 30° C. Sa teneur en oxygène avant et après frittage est peu différente (1.700 et 1 900 ppm) confirmant la faible interaction entre la poudre et l'air, en relation avec la faible élévation de température qui n'est importante qu'aux points de contact entre les microsphères, où s'opère une fusion locale.

Des mesures de surface développée par la méthode BET au krypton ont donné pour les pièces frittées des valeurs d'environ 170 cm² par gramme. L'intérêt du procédé d'électro-étincelage est de pouvoir réaliser toutes pièces de formes et d'épaisseurs désirées, soit de géométrie simple, par exemple dans le cas de pièces cylindriques destinées à réaliser des prothèses laryngées, soit de géométrie plus complexe, par exemple pour des portions de calotte crânienne, toujours à l'aide de moules de forme adaptée.

On rappellera brièvement que des électrodes doivent être judicieusement placées au travers de la paroi du moule, reliées à un appareil à décharge de condensateur, ce qui permet le passage du courant de décharge et la fusion locale des microsphères dans la région de leurs points de contact, et conduit à la consolidation de l'ensemble de la prothèse.

Selon une caractéristique avantageuse additionnelle de la présente invention, les prothèses métalliques présentent une porosité ouverte qui se caractérise par des espaces intersphéroïdaux présentant une dimension sensiblement égale au tiers du diamètre des poudres, comprise donc entre 50 et 150 µm pour des poudres de 150 à 500 µm. Il s'est, en effet, avéré que de tels espaces intersphéroïdaux permettent l'optimisation de la multiplication cellulaire des fibroplastes, cellules indispensables à la colonisation cellulaire de la prothèse.

Lors de la fabrication de la prothèse métallique selon l'invention, il est essentiel de respecter un équilibre entre une épaisseur maximale compatible avec une colonisation cellulaire de bonne qualité et une épaisseur minimale fixée par les contraintes mécaniques que rencontrera la future prothèse lorsqu'elle sera implantée.

L'homme du métier saura parfaitement déterminer ces paramètres pour éviter toute absence de déformation intempestive et/ou assurer à ladite prothèse la résistance aux chocs appropriée à son utilisation. De façon classique, les prothèses posséderont également les points de fixation nécessaires à réaliser les éventuelles sutures au tissu avoisinant.

Selon un autre aspect, la présente invention vise également l'utilisation de microsphères de titane ou d'alliage à base de titane, obtenues par pulvérisation à l'électrode tournante, pour la fabrication d'une prothèse métallique à porosité ouverte de soutien et/ou de remplacement tissulaire. Avantageusement, dans le cas d'une telle utilisation, les microsphères seront solidarisées entre elles par une opération de frittage par électro-étincelage.

Enfin, la présente invention vise bien évidemment le procédé de préparation de telles prothèses métalliques à porosité ouverte. Ce procédé implique la mise en oeuvre des diverses étapes successives suivantes :
- obtention, par pulvérisation à l'électrode tournante (ou tout autre procédé donnant des sphères avec peu ou pas d'aspérités), d'une poudre constituée par des microsphères métalliques de titane ou d'alliage à base de titane ou d'alliages biocompatibles,
- calibrage éventuel des microsphères par tamisage,
- mise en forme de la prothèse par traitement thermique visant à solidariser entre elles lesdites microsphères au niveau de leurs zones de contact mutuel, dans un moule de forme appropriée.

Comme cela a déjà été évoqué précédemment, dans le cadre d'un tel procédé, la mise en forme de la prothèse peut être réalisée par frittage thermique sous vide ou encore de préférence par une opération de mise en forme obtenue par électro-étincelage dans un moule de forme appropriée équipé des électrodes judicieusement disposées au travers des parois desdits moules.

Les microsphères métalliques peuvent être soumises avant et/ou pendant l'opération de mise en forme à une opération de vibrage, ce qui permet d'obtenir un parfait remplissage du moule, une optimisation des propriétés mécaniques et une porosité plus homogène au travers de l'épaisseur de la prothèse ainsi obtenue.

### Exemples de réalisation :

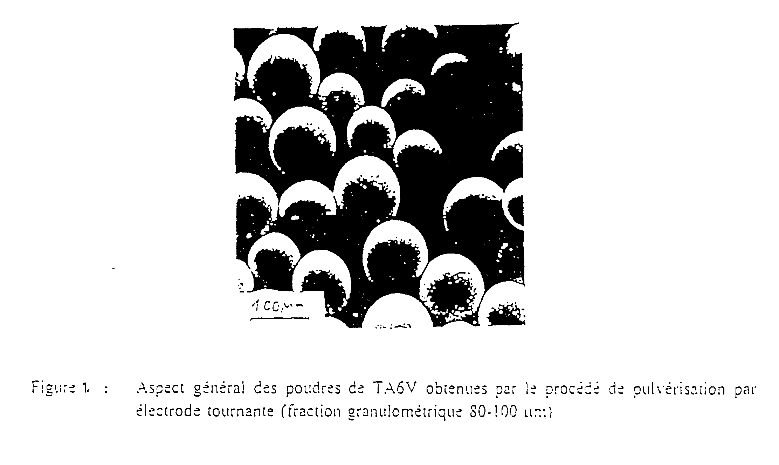

Pour réaliser des pièces de prothèses conformément à la présente invention, le matériau de base utilisé était constitué d'une poudre d'alliage de titane TA6V produite par électrode tournante, de géométrie parfaitement sphérique. On observera que les microsphères représentées à la figure 1 correspondent à une fraction granulométrique à très faible dispersion, comprise entre 80 et 100 µm.

Ces microsphères ont ensuite été placées dans un moule de forme appropriée pour y subir une opération de mise en forme par électro-étincelage.

Le remplissage du moule et l'opération de consolidation doivent être effectués en positionnant la grande dimension de la pièce verticalement afin de favoriser le remplissage et le tassement des microsphères. L'utilisation d'une table vibrante est recommandée pour parfaire le remplissage.

Des plaques planes de 60 mm x 40 mm x 1 mm ont été réalisées. Leur section transverse de passage du courant est de 0,4 cm². L'énergie de décharge utilisée est comprise entre 0,8 et 2,5 kJ. La capacité du banc de condensateurs étant de 80 µF, les tensions de charge variant de 4,5 kV à 8 kV, les densités surfaciques d'énergie se situent donc entre 2 et 5 kJ/cm².

Pour le même procédé, des tubes cylindriques (diamètre intérieur 25 mm - diamètre extérieur 29,3 mm - épaisseur de paroi 2,1 mm - longueur 100 mm) ont été réalisés. Leur section transverse de passage du courant est de 1,8 cm². L'énergie de décharge utilisée est de 15 kJ. La capacité du banc de condensateurs étant de 140 µF, la tension de charge de 146 kV, les densités surfaciques d'énergie sont donc de 8kJ/cm².

Au cours de cette opération, la température globale de la poudre de microsphères n'a jamais dépassé 50°C.

Par cette technique, il a été possible d'obtenir des plaques d'une épaisseur d'un millimètre, découpées en carrés de 10 mm de côté par électroérosion. Ces plaques ont été soumises à des tests de croissance cellulaire et de cytocompatibilité.

Les expérimentations ont notamment été étendues à trois fractions granulométriques, à savoir :
- une fraction granulométrique A inférieure à 125 µm,
- une fraction granulométrique B comprise entre 125 et 160 µm, et
- une fraction granulométrique C comprise entre 160 et 315 µm.

Le matériel biologique a été constitué par des fragments de trachée prélevés sur des embryons de poulet de 16 jours d'incubation.

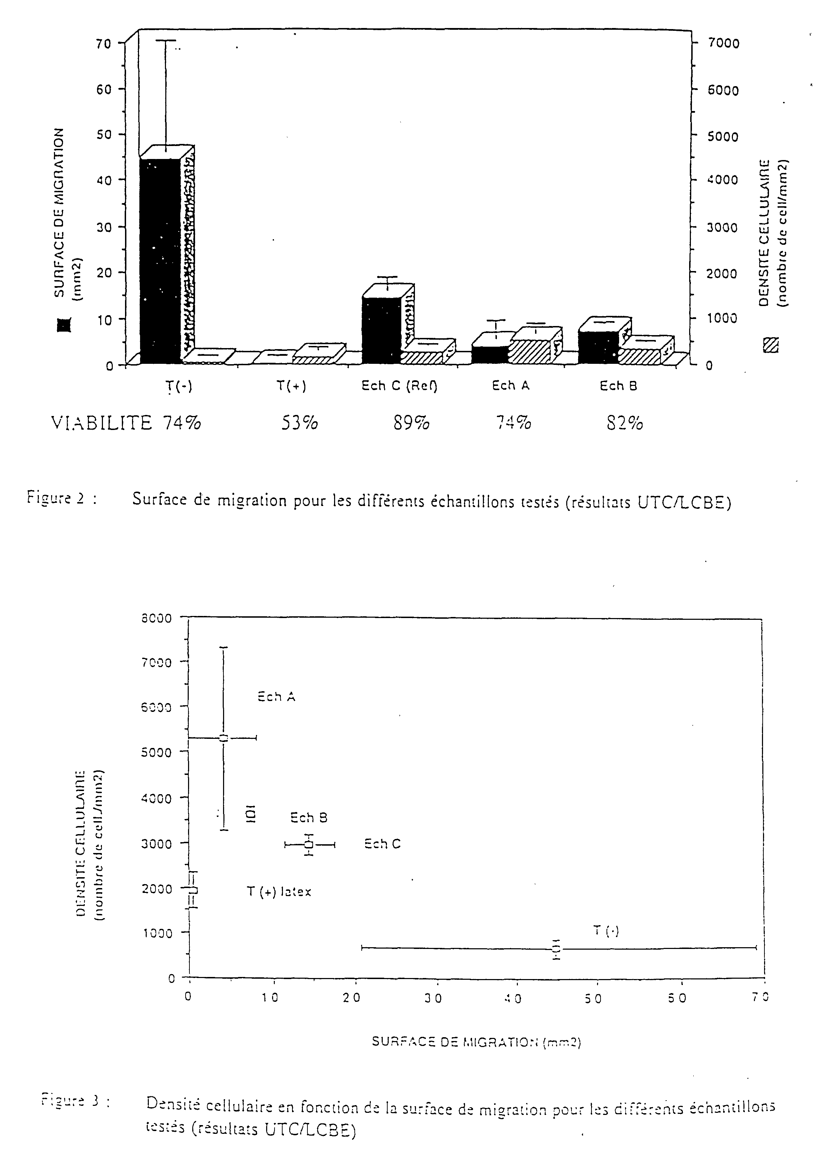

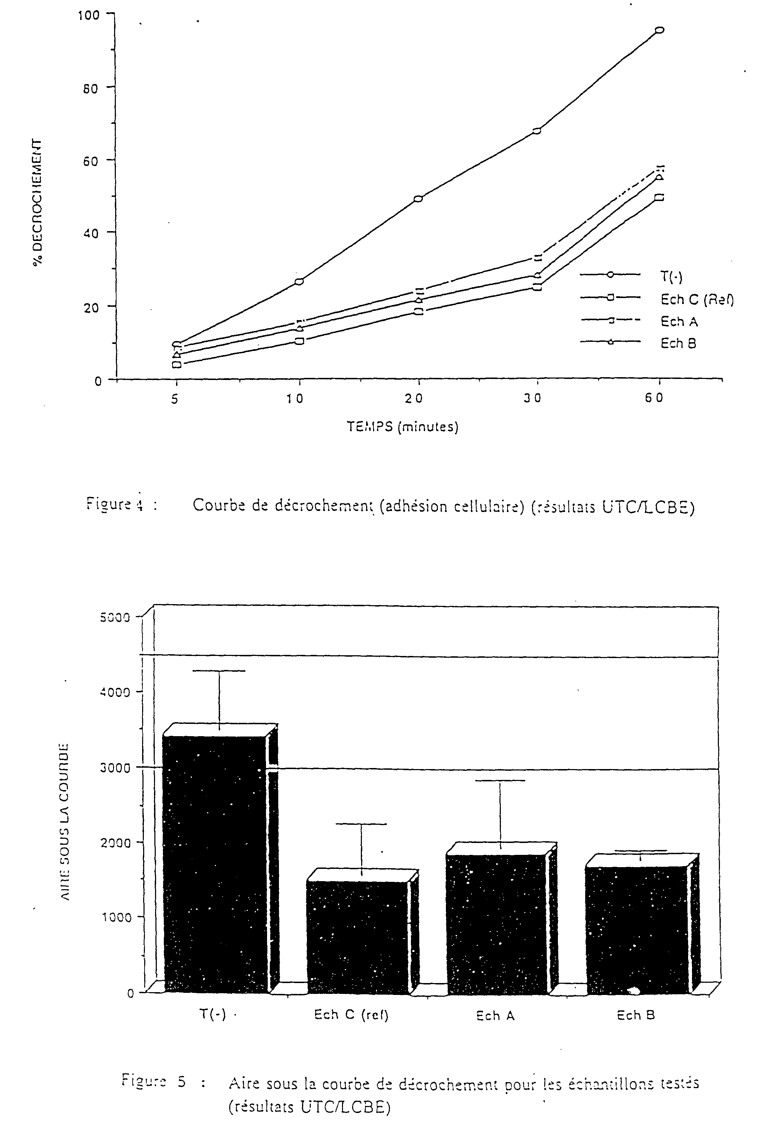

L'échantillon témoin négatif T- (non toxique) est une matière plastique traitée pour culture cellulaire ; l'échantillon témoin positif T⁺ est du latex. Les cultures ont été maintenues 14 jours à 37° C dans le milieu adéquat. Les résultats des tests de multiplication, de migration et d'adhésion cellulaire sont résumés sur les figures 2, 3, 4 et 5 annexées.

L'échantillon témoin négatif T- est une surface sur laquelle se multiplient les cellules sur une seule couche ; la surface de migration est donc importante mais la densité cellulaire quasi-nulle. L'accroche de ces cellules entre elles est donc très faible et leur % de décrochement sera très important.

L'échantillon témoin positif T⁺ ne permet pas (volontairement) la migration des cellules sur une surface plane, mais étudie ponctuellement leur capacité à se joindre entre elles "en boule" sans migrer et sans support. Leur surface de migration est donc nulle mais leur densité non négligeable.

Ces deux échantillons sont dits "témoins".

### Résultats par rapport aux échantillons témoins :

On étudie le meilleur rapport entre la surface de migration dans le biomatériau, la densité cellulaire et la qualité de l'accroche cellulaire.

L'échantillon C (fraction granulométrique la plus importante) a la densité cellulaire la plus faible et la surface de migration la plus importante ; ceci s'explique en raison du volume important que doivent coloniser entre les sphères les fibroblastes, la multiplication cellulaire n'est pas freinée par les obstacles représentés par les microsphères.

L'échantillon A possède la plus forte densité cellulaire, le potentiel de migration des fibroblastes étant ralenti par le contournement des microsphères.

Lors de cette étude expérimentale, il est apparu que dans les conditions utilisées, les échantillons A, B et C présentaient une excellente cytocompatibilité pour le tissu trachéal d'embryon de poulet. Ils ont permis une migration moyenne et une forte adhésion de ces cellules à leur surface. On observe notamment que cette migration est due au fait que les cellules se développent à l'intérieur du matériau à porosité ouverte et le traversent jusqu'à accéder à l'autre face.

Sur les trois échantillons étudiés, la qualité d'accrochage des cellules entre elles n'est pas modifiée par la taille des pores. On a donc tout avantage à choisir un échantillon sur lequel la surface de migration des cellules est la plus rapide.

L'échantillon C est celui qui répond le mieux aux contraintes qu'apportera l'implantation du biomatériau (fraction granulométrique comprise entre 160 et 315 µm).

On atteint ainsi le but recherché, c'est-à-dire une colonisation complète et homogène en volume du matériau de la prothèse par les cellules et la reconstitution d'un organe à l'aide d'une structure poreuse servant de support.

## Revendications

1. Procédé de fabrication de prothèses métalliques à porosité ouverte de soutien et/ou de remplacement tissulaire, notamment pour implantation cervico-maxillo-faciale, en particulier pour reconstitution laryngée, par mise en oeuvre de microsphères de titane ou d'alliage à base de titane ou de tout alliage biocompatible, **caractérisé en ce que** lesdites microsphères qui se présentent sous la forme d'une poudre obtenue par pulvérisation à l'électrode tournante sont placées dans un moule de forme appropriée, puis sont solidarisées entre elles par fusion locale au voisinage de leur point de contact par frittage par électro-étincelage.

2. Procédé selon la revendication 1, **caractérisé en ce que** ledit moule est disposé sur une table vibrante pour favoriser le tassement et le remplissage des microsphères dans ledit moule.

3. Procédé selon l'une des revendications 1 à 2, **caractérisé en ce que** lesdites microsphères présentent une faible dispersion granulométrique comprise entre 50 et 500 µm et, de préférence, entre 150 et 315 µm et **en ce que** les espaces intersphéroïdaux présentent une dimension moyenne comprise entre 50 et 150 µm.

4. Procédé selon l'une des revendications 1 à 3, **caractérisé en ce que** lesdites microsphères sont réalisées en un alliage à base de titane et pouvant contenir au moins un autre métal choisi parmi l'indium, l'étain, le niobium, le palladium, le zirconium, le tantale, le chrome, l'or et le silicium.

## Patentansprüche

1. Verfahren zur Herstellung offenporiger metallischer Prothesen zur Unterstützung und/oder zum Austausch von Gewebe, vor allem für Implantationen im zerviko-maxillo-fazialen Bereich, insbesondere für Rekonstruktionen im Bereich des Kehlkopfs, durch den Einsatz von Mikrokugeln aus Titan oder aus Legierungen auf Titanbasis oder aus jeder biokompatiblen Legierung, **dadurch gekennzeichnet, daß** die Mikrokugeln, die in Form eines durch Pulverisieren mittels einer rotierenden Elektrode erhaltenen Pulvers vorliegen, in eine Form geeigneter Gestalt gebracht werden und dann durch lokales Schmelzen in der Umgebung ihres Kontaktpunkts mittels Sintern durch Funkenerosion miteinander verbunden werden.

2. Verfahren nach Anspruch 1, **dadurch gekennzeichnet, daß** die Form auf einen Rütteltisch gestellt wird, um das Setzen und Auffüllen der Mikrokugeln in der Form zu unterstützen.

3. Verfahren nach einem der Ansprüche 1 bis 2, **dadurch gekennzeichnet, daß** die Mikrokugeln eine geringe Dispersion der Korngröße aufweisen, die zwischen 50 und 500 µm und vorzugsweise zwischen 150 und 315 µm liegt, und dadurch, daß die Zwischenräume zwischen den Kugeln eine mittlere Abmessung aufweisen, die zwischen 50 und 150 µm liegt.

4. Verfahren nach einem der Ansprüche 1 bis 3, **dadurch gekennzeichnet, daß** die Mikrokugeln aus einer Legierung auf Titanbasis hergestellt sind und wenigstens ein anderes Metall enthalten können, das aus Indium, Zinn, Niob, Palladium, Zirkonium, Tantal, Chrom, Gold oder Silizium gewählt ist.

## Claims

1. Method for production of open-pore metallic prostheses for support and/or replacement of tissue, in particular for cervico-maxillo-facial implantation, especially for laryngeal reconstruction, using microspheres of titanium or of titanium-based alloy or of any biocompatible alloy, **characterized in that** said microspheres, which are in the form of a powder obtained by spraying with a rotating electrode, are placed in a suitable mould, then consolidated by local fusion in proximity to their point of contact by electrical discharge sintering.

2. Method according to Claim 1, **characterized in that** said mould is arranged on a vibrating table in order to promote the settling and filling of the microspheres in said mould.

3. Method according to either of Claims 1 and 2, **characterized in that** said microspheres have a low particle size distribution of between 50 and 500 µm, and preferably of between 150 and 315 µm, and **in that** the spaces between the spheres have an average dimension of between 50 and 150 µm.

4. Method according to one of Claims 1 to 3, **characterized in that** said microspheres are made of a titanium-based alloy which can contain at least one other metal chosen from among indium, tin, niobium, palladium, zirconium, tantalum, chromium, gold and silicon.
